Europäisches Patentamt

(19) European Patent Office

Office européen des brevets

(11) Numéro de publication : **0 459 888 A1**

# (12) DEMANDE DE BREVET EUROPEEN

(21) Numéro de dépôt : 91401367.7

(22) Date de dépôt : 28.05.91

(51) Int. Cl.⁵ : **G06F 15/20**

(30) Priorité : 30.05.90 FR 9006728

(43) Date de publication de la demande :
**04.12.91 Bulletin 91/49**

(84) Etats contractants désignés :
**DE GB SE**

(71) Demandeur : **FRANCE TELECOM**
**Etablissement autonome de droit Public**
**(Centre National d'Etudes des**
**Télécommunications), 38/40 rue du Général**
**Leclerc**
**F-92131 Issy les Moulineaux (FR)**
Demandeur : **ASSISTANCE PUBLIQUE,**
**HOPITAUX DE PARIS**
**3, avenue Victoria**
**F-75004 Paris (FR)**

(72) Inventeur : **Bernard, Marc**
**13, rue Gandon**
**F-75013 Paris (FR)**
Inventeur : **Bouchoucha, Michel**
**53, rue Berzélius**
**F-75017 Paris (FR)**

(74) Mandataire : **Mongrédien, André et al**
**c/o BREVATOME 25, rue de Ponthieu**
**F-75008 Paris (FR)**

(54) **Procédé d'analyse automatique de signaux par segmentation et classification.**

(57)    Pour effectuer la segmentation du signal, on procède par itérations, en aggrégeant des échantillons et en fusionnant les impulsions proches. Une fois la segmentation obtenue, on effectue une classification.
Application dans l'analyse des signaux d'origine physiologique.

EP 0 459 888 A1

FIG. 2

La présente invention a pour objet un procédé d'analyse automatique de signaux par segmentation et classification.

L'invention trouve une application privilégiée dans le domaine biomédical, notamment dans le traitement des signaux d'origine physiologique. La première application visée est l'analyse des signaux de contraction oesophagiens. Mais le procédé peut très bien être appliqué à tout signal impulsionnel et bruité.

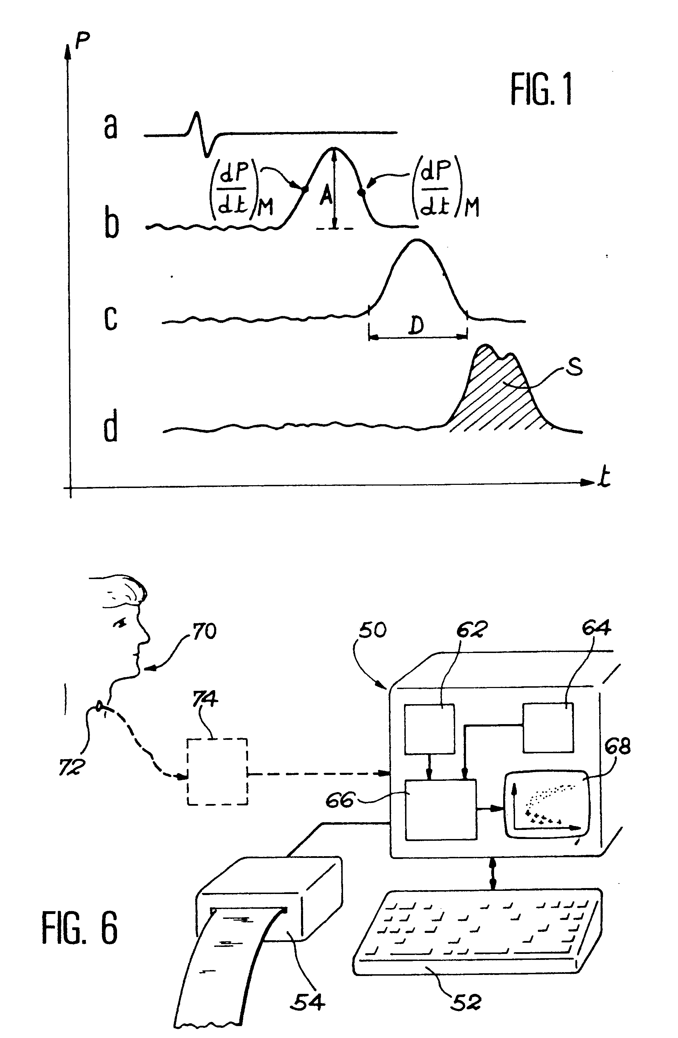

La figure 1 annexée rappelle la forme des ondes péristaltiques à l'origine des signaux à traiter (dans l'application visée).

En (a), on voit le phénomène de déglutition qui déclenche une variation de pression (P) donnant naissance d'abord à une onde de proximité (b) qui se déplace et se déforme (c, d).

L'étude des signaux de contraction oesophagiens amène à mesurer diverses grandeurs comme l'amplitude A du pic, la durée D de l'impulsion, les pentes maximales des fronts de montée ou de descente (dP/dt)M, l'aire sous la courbe S, etc...

Des analyses statistiques sont également entreprises pour obtenir le nombre et les pourcentages des différents types d'impulsions apparaissant dans l'ensemble des signaux mesurés (ondes péristaltiques, tertiaires, polyphasiques, etc...).

L'ensemble de ces résultats présente une signification pour le clinicien.

L'entité physique à traiter (pression, PH, etc...) est mesurée par diverses sondes (oesophagiennes, intestinales, de PHmétrie) ou divers capteurs. Les signaux traduisant cette entité physique sont ensuite amplifiés et enregistrés. Avec l'avénement de l'ordinateur, le dépouillement des mesures ne se fait plus manuellement, mais par des procédés de traitement informatique. Pour cela, le signal est échantillonné et sauvegardé dans un ordinateur muni de moyens de visualisation (écran) ou d'impression.

Cette technique est décrite notamment dans "Computer-Aided Analysis of Human Esophageal Peristalsis-(I) Technical Description and Comparison with Manual Analysis" de Donald O. CASTELL et al. publié dans la revue "Digestive Diseases and Sciences", vol. 29, n° 1, January 1984, pp. 65-72 et dans "Computer Analysis of Human Esophageal Peristalsis and Lower Esophageal Sphincter Pressure.(II) An Interactive System for On-Line Data Collection and Analysis" de June A. CASTELL et al, publié dans "Digestive Diseases and Sciences", vol. 31, n° 11, November 1986, pp. 1211-1216.

Dans de tels procédés de traitement, chaque impulsion est définie non pas par l'ensemble des points qui la constitue mais par quelques paramètres, comme les échantillons de début et de fin et l'échantillon de pic (ou de sommet), tous échantillons définis par leur amplitude et leur instant d'apparition.

Les deux articles cités montrent essentiellement que le recours à l'ordinateur est fondé et fiable, dans la mesure où les résultats obtenus sont en accord très satisfaisant avec les résultats obtenus manuellement.

Cette technique, bien que marquant un progrès, présente encore des inconvénients, notamment en ce qu'elle est semi-automatique et nécessite l'intervention du praticien en cours d'analyse pour initialiser le processus (principalement au commencement de l'analyse). La nature foncièrement non stationnaire des phénomènes étudiés complique singulièrement la tâche.

Le but de l'invention est précisément de remédier à cet inconvénient en proposant un procédé d'analyse entièrement automatique permettant de décharger le médecin du travail fastidieux du repérage des signaux significatifs. Non seulement le procédé de l'invention est entièrement automatique, mais il ne nécessite que très peu d'informations a priori sur le signal, ce qui le rend particulièrement robuste pour des applications dans le domaine médical.

Le procédé de l'invention se divise essentiellement en deux parties : segmentation et classification :

  – pour la segmentation, on détecte les impulsions liées au phénomène étudié à l'aide d'une méthode de croissance d'impulsions utilisant des opérateurs de fusion mettant en oeuvre des critères liés à la forme du signal ;

  – pour la classification, l'ensemble des impulsions obtenues est classé par mise en oeuvre de méthodes d'analyse de données permettant de séparer les parties significatives du bruit.

Dans la première partie du procédé, relative à la détection des impulsions par segmentation, on opère par itérations : on effectue une première segmentation en fixant des seuils arbitraires pour les opérations de fusions d'impulsions. On obtient alors une sur-segmentation du signal. A partir du résultat obtenu, on se fixe de nouveaux seuils et l'on réitère la segmentation ; on procède ainsi jusqu'à ce que le nombre de fusions ne croisse plus. Le signal finalement obtenu est alors soumis à la seconde partie du traitement, à savoir la classification.

De toute façon, les caractéristiques et avantages de l'invention apparaîtront mieux à la lumière de la description qui va suivre. Cette description porte sur des exemples de réalisation donnés à titre explicatif et nullement limitatif et elle se réfère à des dessins annexés, sur lesquels :

  – la figure 1, déjà décrite, montre l'allure des signaux à traiter ;

  – la figure 2 montre diverses étapes dans l'opération de segmentation ;

  – la figure 3 illustre la forme d'un histogramme obtenu après segmentation ;

  – la figure 4 montre schématiquement un exemple de nuage de points finalement obtenu ;

  – la figure 5 est un organigramme général du procédé de l'invention ;

  – la figure 6 illustre une installation permettant la mise en oeuvre du procédé de l'invention.

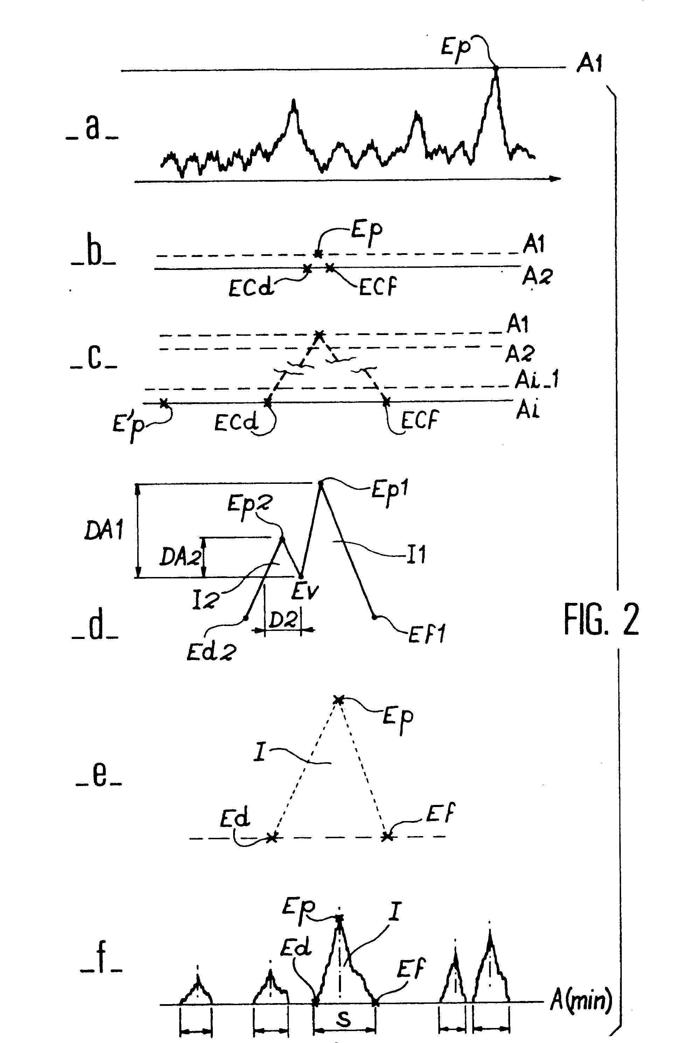

La première phase du procédé de l'invention, destinée à segmenter le signal en tronçons correspondants chacun à une impulsion, est illustrée par la figure 2 (lignes (a) à (e)).

a) Sur la ligne (a) de la figure 2 on voit une partie d'un signal impulsionnel enregistré. On se fixe tout d'abord une amplitude d'analyse ayant une valeur A1 égale à l'amplitude maximum des échantillons mémorisés. L'échantillon correspondant Ep est pris comme échantillon unique marquant à la fois le début, le pic et la fin d'une première impulsion à constituer.

b) On abaisse ensuite l'amplitude d'analyse d'une quantité déterminée et l'on recherche les échantillons mémorisés ayant la nouvelle amplitude abaissée A2, ligne (b).

c) Pour chaque échantillon trouvé (il y en a deux, en l'occurrence soit, ECd et ECf), on détermine si l'échantillon est connexe à l'échantillon Ep déjà trouvé dans l'opération précédente a) et :

c1) si l'échantillon est connexe, il est pris comme nouvel échantillon de début ECd ou de fin ECf de la première impulsion en cours de constitution,

c2) si l'échantillon n'est pas connexe, il est pris comme nouvel échantillon de début, de pic et de fin d'une nouvelle impulsion à constituer.

d) on réitère les opérations b) et c) en abaissant progressivement l'amplitude d'analyse A2,..., Ai-1, Ai (ligne c). Les échantillons connexes de début et de fin d'impulsion se trouvent ainsi aggrégés peu à peu aux impulsions en cours de constitution. Mais de nouveaux échantillons apparaissent, comme E'p sur la ligne c) ; ils sont pris comme pics de nouvelles impulsions à constituer. On voit donc apparaître peu à peu des impulsions.

e) A chaque itération de l'opération d) deux impulsions I1, I2 en cours de constitution sont fusionnées en une seule (cf ligne d) si :

e1) elles possèdent en commun un échantillon Ev, cet échantillon définissant une vallée entre les deux pics Ep1, Ep2,

e2) ayant calculé pour chaque impulsion I1, I2 la différence entre l'amplitude de l'échantillon de pic Ep1, Ep2 et l'amplitude de l'échantillon commun de vallée Ev, le rapport de ces différences est supérieur à un premier seuil T1,

e3) la durée de l'impulsion ayant la plus petite différence d'amplitude entre pic Ep2 et vallée Ev (dans le cas illustré sur la ligne d, c'est l'impulsion I2) est inférieure à un deuxième seuil T2,

e4) les deux différences d'amplitude pic-vallée sont inférieures à un troisième seuil T3.

La fusion de deux impulsions I1, I2 en une impulsion unique I (cf ligne e) consiste à prendre, pour échantillon de début de l'impulsion unique, l'échantillon de début Ed de la première impulsion I2, pour échantillon de fin, l'impulsion de fin Ef de la seconde impulsion I1 et pour échantillon de pic, l'échantillon de pic Ep de la plus haute des deux impulsions I1.

Dans cette opération de fusion, les trois seuils T1, T2, T3 ont, dans cette première phase (A), trois valeurs arbitraires.

f) On abaisse ainsi l'amplitude d'analyse jusqu'à atteindre une valeur minimale prédéterminée A(min) (ligne f), ce qui laisse alors, à la dernière itération correspondant à cette valeur minimale, une suite de segments S définissant chacun une impulsion I avec un échantillon de pic Ep, un échantillon de début Ed et un échantillon de fin Ef.

Ayant obtenu cette première segmentation, à partir de trois seuils choisis arbitrairement, on va affiner la segmentation en modifiant ces seuils. Trois nouveaux seuils vont être définis à partir des résultats obtenus à la suite de la première segmentation. Pour cela, on effectue une analyse statistique des impulsions obtenues (ligne f de la figure 2) pour établir trois histogrammes, relatifs respectivement aux différences pic-vallée, aux durées et aux amplitudes des impulsions obtenues.

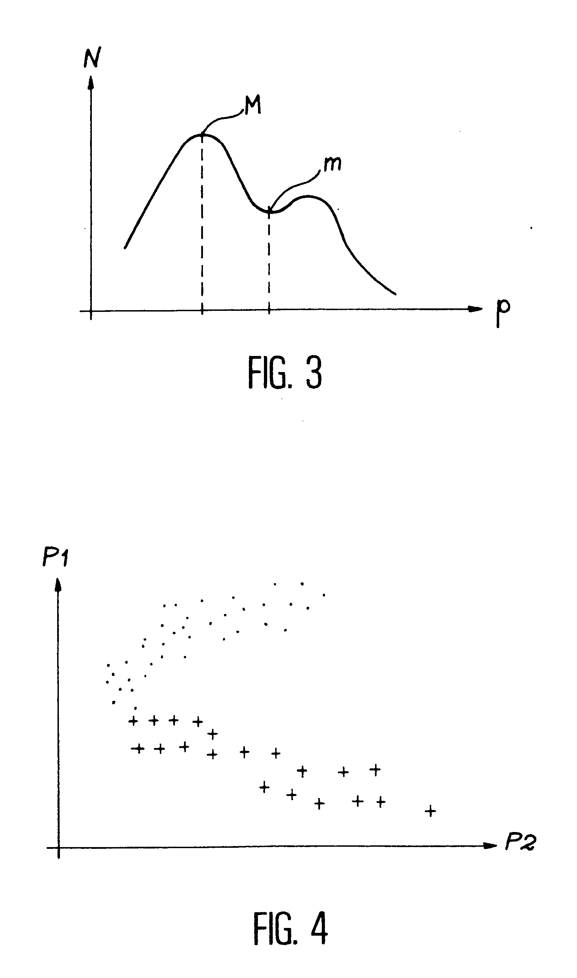

Ces histogrammes ont l'allure de la courbe de la figure 3 qui représente le nombre N d'impulsions ayant pour paramètre p une certaine valeur. Un premier maximum M correspond aux impulsions de bruit. Il est suivi d'un minimum m qui donne la valeur du nouveau seuil à prendre pour le paramètre en question (différence pic-vallée, durée, amplitude).

On réitère alors les opérations d) à f) de la première phase avec les nouveaux seuils T'1, T'2, T'3 définis, ce qui permet de resegmenter le signal en refusionnant de nouvelles impulsions.

On réitère les opérations d) à f) de la première phase, et ainsi de suite, jusqu'à ce que le traitement ne permette plus d'effectuer de nouvelles fusions d'impulsions et ne réduise plus le nombre d'impulsions obtenues. Le signal est alors définitivement segmenté et la série d'impulsions obtenue est celle sur laquelle l'analyse peut alors être entreprise.

Cette opération de segmentation définit une série d'impulsions caractérisées chacune par leur début, leur fin et leur pic. Mais seul un sous-ensemble de ces impulsions est significatif, c'est-à-dire correspond à des signaux de contraction oesophagiens. Les autres impulsions correspondent au bruit dû à la respiration, aux battements cardiaques et à la toux du patient. Il est donc nécessaire de poursuivre l'analyse du signal par une classification permettant de séparer les impulsions significatives du bruit.

De préférence, on met en oeuvre une méthode dite des nuées dynamiques. Pour cela, on définit quantitativement chaque impulsion par des paramètres comme :

– la différence entre l'amplitude du pic et l'amplitude de l'extrémité la plus basse,

– la largeur de l'impulsion,

– l'amplitude moyenne de l'impulsion,

– la largeur de l'impulsion à l'amplitude moyenne,

– la variance de l'amplitude,

– la moyenne des pentes du front de montée et du front de descente de l'impulsion,

– la moyenne des différences entre l'amplitude d'un pic et les amplitudes des deux pics voisins.

On représente l'ensemble des impulsions obtenues dans un espace à autant de dimensions que de paramètres choisis, ce qui conduit à un nuage de points dans cet espace, chaque point correspondant à une impulsion.

Dans le cas où les sept paramètres précédents sont retenus, on obtient un nuage de points dans un espace à sept dimensions.

De préférence, on partitionne le nuage de points obtenus en deux classes, la première correspondant aux impulsions significatives plutôt courtes et de grande amplitude et la seconde correspondant aux impulsions plutôt longues et de faible amplitude.

Selon une autre variante, on partitionne le nuage de points obtenu en trois classes, la première correspondant aux impulsions de forte amplitude, la seconde correspondant aux impulsions de très longue durée, la troisième correspondant aux impulsions intermédiaires, et l'on partitionne ensuite la troisième classe en deux sous-classes.

De préférence encore, pour adapter le poids relatif de chaque paramètre aux caractéristiques spécifiques de chaque enregistrement, on effectue sur le nuage de points obtenu, une analyse en composante principale, ce qui permet d'analyser le nuage de points selon ses axes principaux. Ce traitement permet de séparer plus nettement les différentes classes rencontrées.

La figure 4 illustre schématiquement l'allure du nuage de points affiché sur un écran d'un calculateur après le traitement qui vient d'être décrit. Le nuage correspond à la distribution des impulsions selon deux paramètres P1, P2. Les croix sont censées représenter les impulsions non significatives et les points les impulsions significatives.

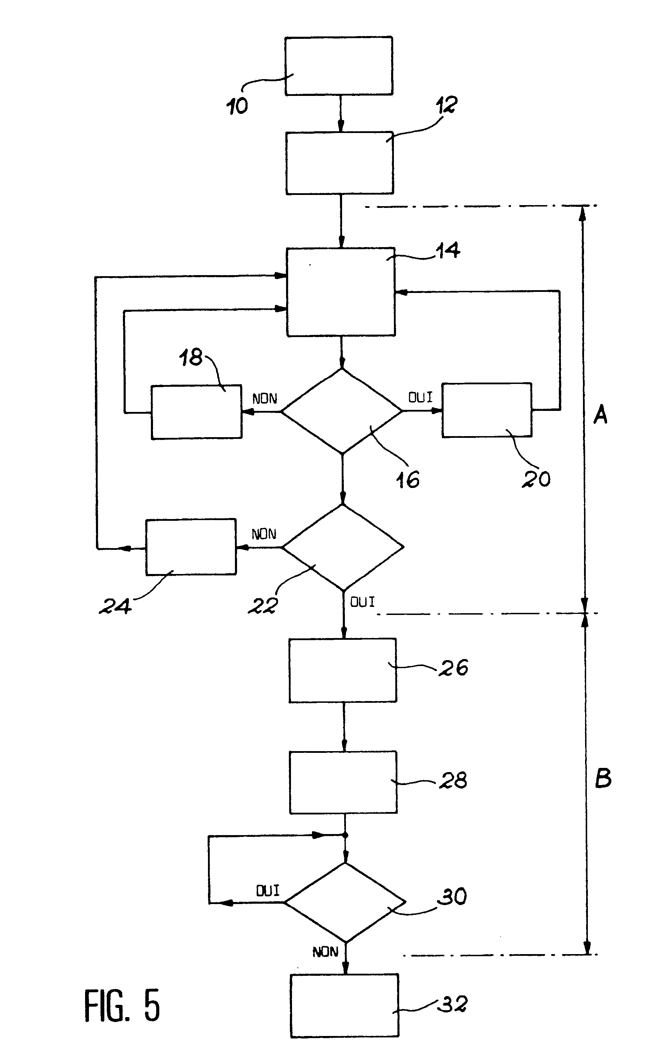

La figure 5 montre l'organigramme général du procédé qui vient d'être décrit. La signification des blocs représentés est la suivante :

10 : déterminer l'amplitude maximum (A1) et l'amplitude minimum (Amin),

12 : donner à l'amplitude d'analyse A la valeur du maximum,

Phase A :

14 : opération de croissance des impulsions,

16 : y-a-t-il connexité ?

18 : s'il n'y a pas connexité, formation d'une nouvelle impulsion,

20 : s'il y a connexité, aggrégation du nouvel échantillon à l'impulsion en cours de croissance,

22 : l'amplitude d'analyse est-elle égale à l'amplitude minimum ?

24 : si la réponse à la question 22 est négative, faire A=A-1, c'est-à-dire diminuer l'amplitude d'analyse,

Phase B :

26 : si la réponse à la question 22 est affirmative, effectuer l'analyse statistique des impulsions obtenues,

28 : fixer de nouveaux seuils,

30 : des impulsions ont-elles été fusionnées depuis la dernière itération ? Dans l'affirmative, on réitère les opérations de fusion d'impulsions avec les nouveaux seuils, dans la négative, l'analyse est terminée,

32 : classification des impulsions.

La figure 6, enfin, montre schématiquement un dispositif permettant de mettre en oeuvre le procédé qui vient d'être décrit. Ce dispositif comprend un calculateur 50 (qui peut être un calculateur de type PC), un clavier 52 et une imprimante 54. Le calculateur comprend une mémoire 62 contenant les données à traiter (sous forme de disquette par exemple), une mémoire 64 de type ROM contenant le programme de traitement, une unité centrale de traitement 66 et un écran 68.

La figure 6 montre encore un patient 70 sur l'oesophage duquel on a posé une sonde 72 capable de mesurer la pression. Un amplificateur 74 permet de délivrer un signal électrique qui est ensuite échantillonné puis converti en numérique et stocké dans la mémoire 62 du calculateur.

Naturellement, comme le procédé qui vient d'être décrit s'effectue en différé, on peut enregistrer préalablement le signal sur un support approprié (disquette par exemple), indépendamment de tout calculateur, puis introduire, le moment voulu, ce support dans un calculateur, pour effectuer le traitement du signal.

**Revendications**

1. Procédé d'analyse automatique d'un signal impulsionnel, notamment d'origine physiologique, dans lequel on mesure une entité physique, on convertit cette entité en données numériques que l'on mémorise et que l'on traite en différé pour en extraire des impulsions significatives et pour obtenir certaines caractéristiques statistiques de l'entité physique mesurée, ce procédé étant caractérisé par le fait que, le traitement destiné à extraire les impulsions significatives comprend les opérations suivantes :

A) dans une première phase : on définit, dans le signal, des segments correspondant chacun à une impulsion ayant un échantillon de début, marquant une extrémité du segment, un échantillon de pic marquant le sommet de l'impulsion et un échantillon de fin marquant l'autre extrémité du segment, cette première phase dite de segmentation comprenant les opérations suivantes :

a) on fixe une amplitude d'analyse de valeur (A1) égale à l'amplitude maximum des échantillons mémorisés, l'échantillon correspondant (Ep) étant pris comme échantillon de début, de pic et de fin d'une première impulsion à constituer,

b) on abaisse l'amplitude d'analyse d'une quantité déterminée et l'on recherche les échantillons mémorisés ayant cette nouvelle amplitude abaissée (A2),

c) pour chaque échantillon trouvé, on détermine s'il est connexe à l'échantillon déjà trouvé dans l'opération précédente a) et :

c1) si l'échantillon est connexe, il est pris comme nouvel échantillon de début (ECd) ou de fin (ECf) de l'impulsion en cours de constitution ,

c2) si l'échantillon n'est pas connexe, il est pris comme nouvel échantillon de début, de pic et de fin d'une nouvelle impulsion à constituer,

d) on réitère les opérations b) et c) en abaissant progressivement l'amplitude d'analyse (A1,...Ai-1, Ai), ce qui aggrège peu à peu des échantillons connexes de début et de fin d'impulsion aux impulsions en cours de constitution et fait apparaître de nouveaux échantillons pris comme pics de nouvelles impulsions à constituer,

e) à chaque itération de l'opération d), deux impulsions (I1, I2) en cours de constitution sont fusionnées en une seule si :

e1) elles possèdent en commun un échantillon (Ev) définissant une vallée entre les deux pics (Ep1, Ep2) des deux impulsions (I1, I2),

e2) ayant calculé pour chaque impulsion la différence entre l'amplitude de l'échantillon de pic (Ep1, Ep2) et l'amplitude de l'échantillon commun de vallée (Ev), le rapport de ces différences est supérieur à un premier seuil (T1),

e3) la durée (D2) de l'impulsion (I2) ayant la plus petite différence d'amplitude entre pic (Ep2) et vallée (Ev) est inférieure à un deuxième seuil (T2),

e4) les deux différences d'amplitude pic-vallée sont inférieures à un troisième seuil

(T3),

la fusion de deux impulsions en une impulsion unique consistant à prendre, pour échantillon de début de l'impulsion unique, l'échantillon de début (Ed) de la première impulsion, pour échantillon de fin, l'impulsion de fin (Ef) de la seconde impulsion et pour échantillon de pic, l'échantillon de pic (Ep) de la plus haute des deux impulsions, les trois seuils (T1, T2, T3) ayant, dans cette première phase, trois valeurs arbitraires,

f) on abaisse l'amplitude d'analyse jusqu'à atteindre une valeur minimale prédéterminée (Amin), ce qui laisse alors, à la dernière itération correspondant à cette valeur minimale, une suite de segments (S) définisssant chacun une impulsion avec une amplitude de pic, une durée et une différence pic-vallée,

B) dans une seconde phase :

g) on effectue une analyse statistique des impulsions obtenues après la première phase de segmentation et l'on détermine trois histogrammes relatifs respectivement aux différences pic-vallée, aux durées et aux amplitudes des impulsions obtenues,

h) à partir des trois histogrammes obtenus, on définit trois nouveaux seuils respectivement pour le rapport des différences pic-vallée (T'1), pour la durée (T'2) et pour les différences pic-vallée (T'3),

i) on réitère l'opération e) de la première phase avec ces nouveaux seuils (T'1, T'2, T'3), ce qui permet de resegmenter le signal en reconstituant et en refusionnant de nouvelles impulsions,

j) avec les nouvelles impulsions obtenues après l'opération i), on réitère l'opération e) de la première phase, et ainsi de suite, jusqu'à ce que le traitement ne permette plus d'effectuer de nouvelles fusions d'impulsions et ne réduise plus le nombre d'impulsions obtenues, ce qui laisse un signal définitivement segmenté avec une série définitive d'impulsions sur laquelle l'analyse est alors entreprise.

2. Procédé selon la revendication 1, caractérisé par le fait qu'il comprend, après la deuxième phase, une opération de classification des impulsions, dans laquelle :

a) on définit quantitativement chaque impulsion par des paramètres comme :

- la différence entre l'amplitude du pic et l'amplitude de l'extrémité la plus basse,
- la largeur de l'impulsion,
- l'amplitude moyenne de l'impulsion,
- la largeur de l'impulsion à l'amplitude moyenne,
- la variance de l'amplitude,

– la moyenne des pentes du front de montée et du front de descente de l'impulsion,
– la moyenne des différences entre l'amplitude d'un pic et les amplitudes des deux pics voisins,
b) on représente l'ensemble des impulsions obtenues dans un espace à autant de dimensions que de paramètres choisis, ce qui conduit à un nuage de points dans cet espace, chaque point correspondant à une impulsion et on analyse le nuage obtenu.

3. Procédé selon la revendication 2, caractérisé par le fait qu'on partitionne le nuage de points obtenu en deux classes, la première correspondant aux impulsions significatives plutôt courtes et de grande amplitude et la seconde correspondant aux impulsions plutôt longues et de faible amplitude.

4. Procédé selon la revendication 2, caractérisé par le fait qu'on partitionne d'abord le nuage de points obtenu en trois classes, la première correspondant aux impulsions de forte amplitude, la seconde correspondant aux impulsions de très longue durée, la troisième correspondant aux impulsions intermédiaires, et qu'on partitionne ensuite la troisième classe en deux sous-classes.

5. Procédé selon la revendication 2, caractérisé par le fait que l'on effectue, sur le nuage de points obtenu, une analyse en composante principale.

FIG. 1

$\left(\dfrac{dP}{dt}\right)_M$

$\left(\dfrac{dP}{dt}\right)_M$

FIG. 6

FIG. 2

FIG. 3

FIG. 4

EP 0 459 888 A1

FIG. 5

11

EP 0 459 888 A1

Office européen
des brevets

**RAPPORT DE RECHERCHE EUROPEENNE**

Numero de la demande

EP    91 40 1367

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | PROCEEDINGS OF PATTERN RECOGNITION 1982, NEW YORK, US pages 988 - 990; B.N. CHATTERJI ET AL.: 'Toward a feature extraction scheme for ECG recognition ' * page 988, colonne 1, ligne 11 - ligne 13 * * colonne 2, ligne 22 - ligne 47 * * page 989, colonne 1, ligne 21 - ligne 23 * * page 989, colonne 1, ligne 44 - ligne 47 * --- | 1-5 | G06F15/20 |
| A | PROCEEDINGS OF THE 8TH INTERNATIONAL CONF. ON PATTERN RECOGNITION 31 Octobre 1986, PARIS, FR pages 380 - 382; E. SKORDALAKIS ET AL.: 'Primitive pattern selection and extraction in ECG waveforms ' * page 380, colonne 2, ligne 17 - ligne 37 * * page 381, colonne 2, ligne 2 - ligne 3 * * page 382, colonne 1, ligne 17 - ligne 36 * --- | 1-5 | |
| A | IEEE TRANSACTIONS ON BIOMEDICAL ENGINEERING vol. BME33, no. 10, Octobre 1986, NEW YORK, US pages 972 - 974; E. SKORDALAKIS: 'Recognition of the shape of the ST segment in ECG waveforms ' ----- | 1-5 | **DOMAINES TECHNIQUES RECHERCHES (Int. Cl.5)**<br><br>G06F |

Le présent rapport a été établi pour toutes les revendications

| Lieu de la recherche | Date d'achèvement de la recherche | Examinateur |
|---|---|---|
| LA HAYE | 23 AOUT 1991 | BARBA M. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

12